# EUROPEAN PATENT APPLICATION

(11) **EP 4 653 577 A1**
(43) Date of publication of application: **26.11.2025**
(21) Application number: 23917250.5
(22) Date of filing: 07.12.2023
(51) Int. Cl.: C25B 1/04, C25B 15/00, C25B 9/00

(54) **HYDROGEN GENERATING DEVICE HAVING EXTRACTABLE FILTERING STRUCTURE**

(30) Priority: 19.01.2023 CN 202310056589
(71) Applicant: Lin, Hsin-Yung, Taoyuan, Taiwan 33341 (TW)
(72) Inventor: Lin, Hsin-Yung, Taoyuan, Taiwan 33341 (TW)
(74) Representative: Wang, Bo
(86) International application number: PCT/CN2023/136921
(87) International publication number: WO 2024/152782

(57) **Abstract**

A hydrogen generator with detachable filter comprises a water tank, an electrolysis module configured in the water tank, a filter channel device coupled to the water tank, a humidifying module, vertically configured above the water tank, an integrated channel device vertically configured above the humidifying module, and a condenser configured on the integrated channel device. The electrolysis module is configured to electrolyze water contained in the water tank to generate gas comprising hydrogen. The humidifying module includes a humidifying chamber and a gas channel isolated from the humidifying chamber. The filtering device is arranged in the gas channel to receive and filter the gas comprising hydrogen generated by the electrolysis module. The condenser is configured to condense the gas comprising hydrogen outputted by the filtering device. The integrated channel device includes a gas input channel for guiding the gas comprising hydrogen outputted from the condenser into the humidifying chamber.

## Description

### BACKGROUND OF THE INVENTION

### 1. Field of the invention

The present invention relates to a hydrogen generator, and more particularly, to a hydrogen generator with detachable filter.

### 2. Description of the prior art

People always pay much attention to human life, so many medical technologies are developed to fight disease and keep people alive. Most of the past medical treatment methods are passive; namely, when a disease occurs, the medical treatment, such as surgery, medication and chemotherapy, radiation or recovery from chronic illness, rehabilitation and correction are given. However, in recent years, many medical experts have increasingly concentrated on preventive medical methods, such as healthy food research, genetic disease screening and disease prevention, and even have actively taken initiatives to prevent possible future diseases. In addition, in order to extend life expectancy of human beings, numerous anti-aging and antioxidant technologies, including skin care products and foods/anti-oxidant drugs, have been gradually developed and widely adopted by the public.

Researches show that the body's unstable oxygen gas (O+), also known as free radicals (harmful free radicals), caused by various reasons (such as disease, diet, environment or living habits), can mix with inhaled hydrogen gas to form water and be excreted by the body. Namely, by indirectly reducing the number of free radicals in the human body, the reduction of acidic physique is achieved to become healthy alkaline physique, which can resist oxidation and anti-aging, as well as achieve the effects of eliminating chronic diseases and beauty care. Moreover, by increasing the amount of hydrogen gas inhaled, the increase of the time spent on inhaling hydrogen gas (for example, inhaling hydrogen gas during sleeping time) may also efficiently improve the effect of hydrogen gas inhalation.

Currently on the market, hydrogen generator usually generates hydrogen by electrolyzing water which contains electrolyte. Further, when the hydrogen generator generates hydrogen gas, the hydrogen gas will entrain steam, and the steam contains a small amount of electrolyte. Therefore, in order to allow users to inhale hydrogen with higher purity, the hydrogen generator of the prior art will include a filter or condensation device to filter electrolytes and impurities in hydrogen gas. However, it leads to poor effect as a result of accumulating a large amount of electrolytes and impurities in the hydrogen generator or the condensation device of the hydrogen generator after long-term use. In addition, when the hydrogen generator generates hydrogen gas, the electrolysis device raises the operating temperature, and further raises the operating temperature in the hydrogen gas. If the condensation device cannot effectively reduce the temperature of hydrogen gas and condense the steam in hydrogen gas, users may inhale residual electrolytes and electrical substances, thereby reducing the effect. It is therefore necessary to clean the filter or condensation device regularly to maintain filtration effect and condensation effect. However, the pipelines of the hydrogen generator of the prior art are closed pipelines and the pipelines are integrated structures, so the hydrogen generator needs to be disassembled to clean filter or condensation device, which is inconvenient and complicated to clean.

Therefore, it is necessary to develop a new type of hydrogen generator to solve the problems of the prior art.

### SUMMARY OF THE INVENTION

Therefore, the present invention provides a hydrogen generator with detachable filter to solve the problems of the prior art.

In one embodiment of the present invention, the hydrogen generator with detachable filter comprises a water tank, an electrolysis module, a humidifying module, a filter channel device, a condenser and an integrated channel device. The water tank has an accommodation space for accommodating an electrolyzed water. The electrolysis module is arranged in the accommodation space of the water tank. The electrolysis module is configured to receive and electrolyze the electrolyzed water from the water tank to generate and output a gas comprising hydrogen. The humidifying module is stacked above the water tank to humidify the gas comprising hydrogen. The humidifying module has a humidifying chamber for accommodating a replenishing water. The filter channel device is coupled with the water tank. The filter channel device comprises a channel case and a filter component in the channel case. The filter channel device is configured to receive and filter the gas comprising hydrogen from the electrolysis module and output the gas comprising hydrogen after filtering. The condenser is stacked above the water tank. The condenser is fluidly coupled to the filter channel device to receive and condense the gas comprising hydrogen from the filter channel device. The integrated channel device is stacked above the water tank. The integrated channel device comprises a gas input channel, fluidly coupling the condenser and the humidifying chamber to guide the gas comprising hydrogen from the condenser into the humidifying chamber. Wherein, the filter channel device, the condenser and the humidifying device are respectively engaged with the integrated channel module. The filter channel device penetrates the humidifying module and the integrated channel module. The filter channel device is detachable from the humidifying module and the integrated channel device.

Wherein, the humidifying module further comprises a gas channel extending upward from the bottom to the top of the humidifying module. The gas channel and the humidifying module are isolated from each other, and the filter channel device passes through the gas channel.

Wherein, the integrated channel device comprises a hole, and the filter channel device passes through the hole.

Wherein, the length of the filter channel device is larger than the length of the hole combined with the gas channel, and the filter channel device penetrates the integrated channel device and directly connects to the condenser.

Wherein, the condenser is detachably engaged with the integrated channel module. The condenser comprises a base engaged with the integrated channel module, a condensation tube detachably engaged with the base, and a heat dissipation element covering the condensation tube.

Wherein, the condenser further comprises a spiral structure configured in the condensation tube to form a condensation flow channel in the condensation tube. The gas comprising hydrogen passes through the condensation tube along the condensation flow channel.

Wherein, the length of the base of the condenser accounts for at least one-half of the side length of the hydrogen generator.

Wherein, the filter channel device further comprises a metal mesh component configured in the channel case.

Wherein, the filter component is a plurality of baffle structures, and the baffle structures are staggered and configured in the channel case to form a filter channel.

Wherein, the each baffle structure comprises an arc part and a bottom hook part connected to the arc part. Wherein, the arc part extends upward and the bottom hook part extends downward from the top of the arc part.

Wherein, the filter channel comprises a S-shaped flow channel.

Furthermore, the hydrogen generator with detachable filter further comprises a fining device in the humidifying chamber and the fining device is fluidly connected to the condenser through the integrated channel device. The fining device is configured to fine and evenly distribute the gas comprising hydrogen from the condenser into the humidifying chamber. Wherein, the fining device further comprises a plurality of micro holes for the gas comprising hydrogen flowing through into the humidifying chamber and forming a plurality of micro bubbles in the replenishing water.

Wherein, the electric potential of the water tank is equal to that of the filter channel device, and the hydrogen generator with detachable filter further comprises a conductive component connected to the filter channel device and extending downward into the water tank.

Wherein, the hydrogen generator with detachable filter further comprises a nebulizer engaged with the integrated channel device. The nebulizer receives the gas comprising hydrogen from the integrated channel device and selectively generates an atomized gas to be mixed with the gas comprising hydrogen to form a health caring gas.

Wherein, the hydrogen generator with detachable filter further comprises an active filter tube penetrating the water tank and engaging with the humidifying module. The active filter tube is fluidly coupled to the humidifying module and the integrated channel device. The active filter tube is detachable from the water tank. The active filter tube is configured to receive and filter the gas comprising hydrogen from the humidifying chamber and output the gas comprising hydrogen after filtering to the integrated channel device.

Wherein, the water tank comprises a tank body and a cover. The tank body comprises a tank channel and the cover comprises a cover channel. The active filter tube penetrates the cover channel and the tank channel to connect to the humidifying module, and the gas comprising hydrogen flows through the cover channel and the tank channel to connect the humidifying module, and one shell of the activated filter tube and the humidifying module form a closed cavity. Wherein, the gas comprising hydrogen flows from the humidifying module to the closed cavity and enters into the activated filter tube, and then flows through the humidifying module to the integrated channel device.

Wherein, the electrolysis module is configured in the accommodation space of the water tank. The water tank comprises a tank body and a cover, and the electrolysis module has an electrolysis tank. The cover includes a first fixing part. The electrolysis module includes a second fixing part toward the cover to be connected to the first fixing part, so as to suspend the electrolysis module from the cover.

Wherein, the cover comprises a first positioning structure, and the electrolysis module comprises a second positioning structure corresponding to the first positioning structure. Wherein, when the cover and the electrolysis module are connected to each other with the first fixing part and the second fixing structure to suspend the electrolysis module from the cover, the first positioning structure is coupled to the second positioning structure.

Wherein, the tank body forms a plurality of third positioning structures at the bottom of the accommodation space, and the bottom of the electrolysis module comprises a plurality of fourth positioning structures corresponding the third positioning structures. When the electrolysis module is configured in the accommodation space, the third positioning structures are respectively coupled with the fourth positioning structures.

In summary, the hydrogen generator with detachable filter of the present invention has the separately filter channel device and the condenser. The user can directly pull out the filter channel device and disassemble the condenser without disassembling other components or devices when the filter channel device and the condenser need to be cleaned or the filter channel device needs to replace, so as to increase convenience and installation efficiency. In addition, the condenser of the hydrogen generator with detachable filter of the present invention can effectively improve the condensation path and heat dissipation function through the extendable condensation flow channel and the heat dissipation element so as to improve condensation and filtration efficiency.

### BRIEF DESCRIPTION OF THE APPENDED DRAWINGS

FIG. 1 is a structural schematic diagram illustrating a hydrogen generator with detachable filter according to an embodiment of the present invention.
FIG. 2 is a functional block diagram illustrating the hydrogen generator with detachable filter of FIG. 1.
FIG. 3 is an exploded diagram illustrating the hydrogen generator with detachable filter of FIG. 1.
FIG. 4A is an exploded diagram illustrating the water tank of FIG. 1.
FIG. 4B is a structural schematic diagram illustrating the humidifying module of FIG. 1.

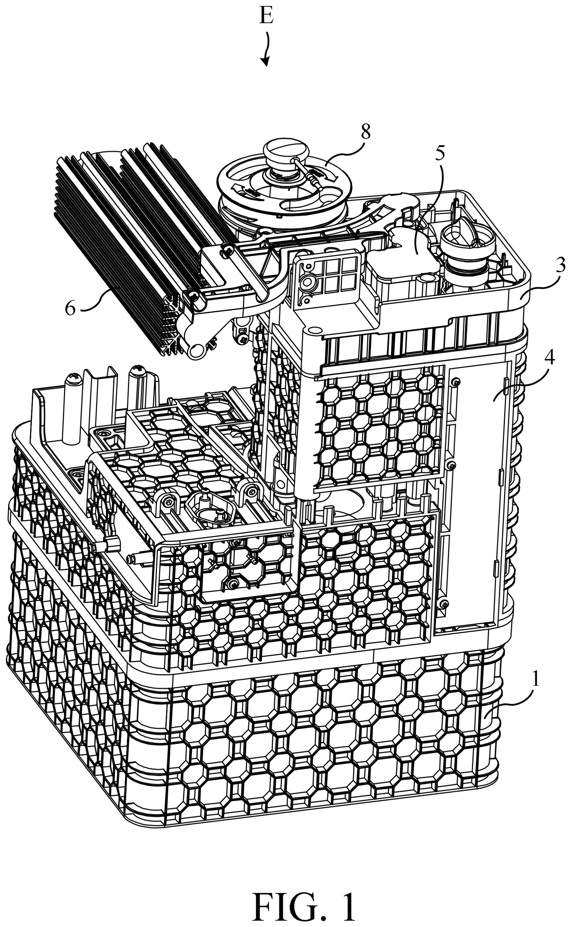

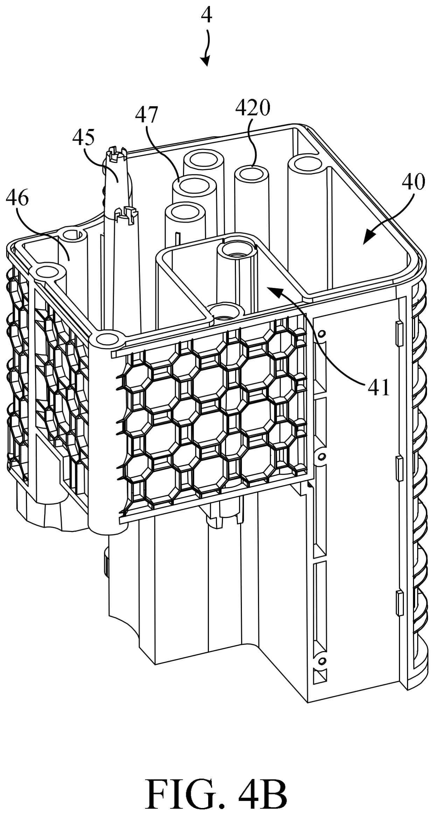

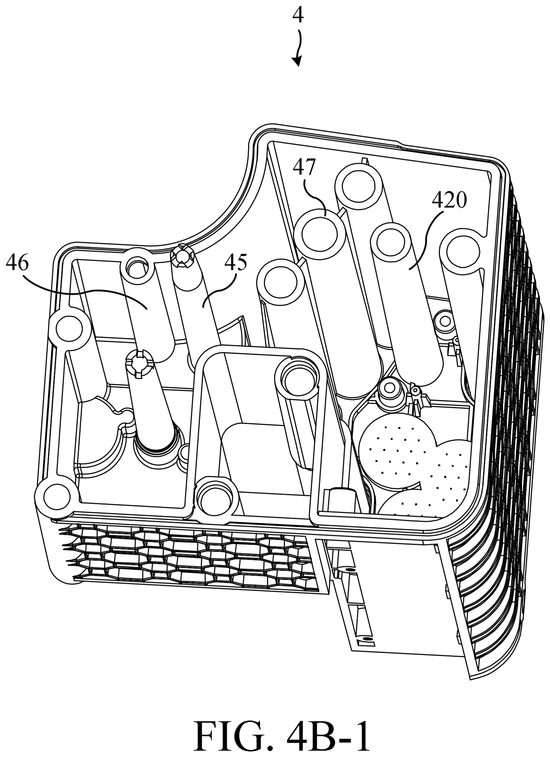

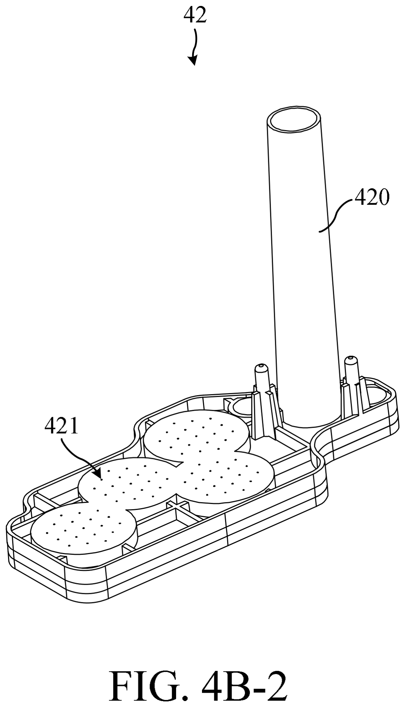

FIG. 4B-1 is a structural schematic diagram illustrating the humidifying module of FIG. 1 in another one perspective.
FIG. 4B-2 is a structural schematic diagram illustrating the fining structure

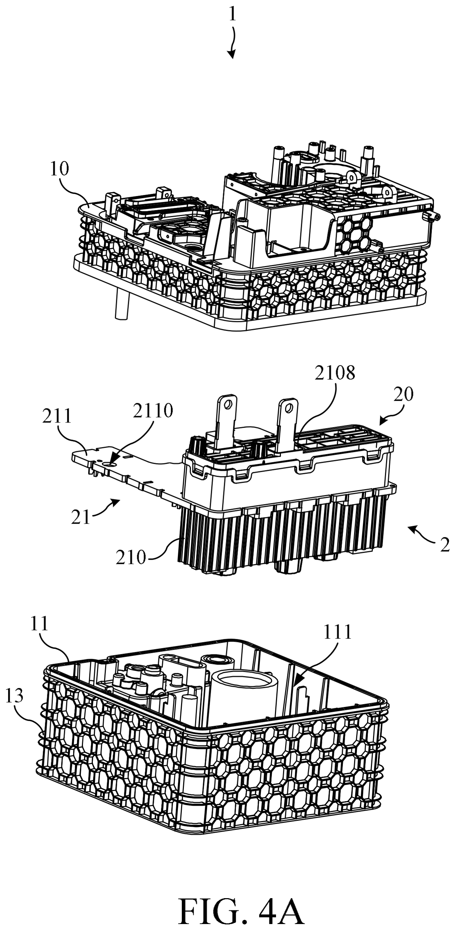

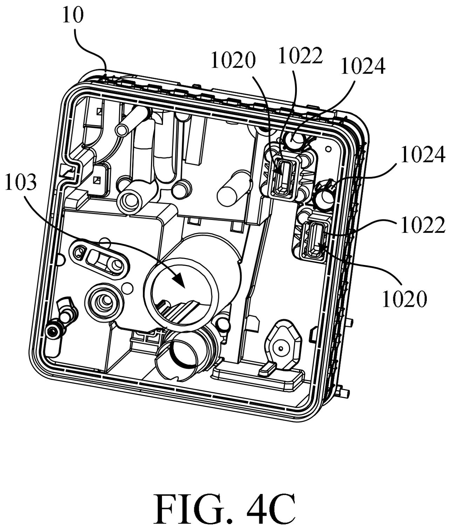

FIG. 4C is a structural schematic diagram illustrating the cover of FIG. 4A in another one perspective.

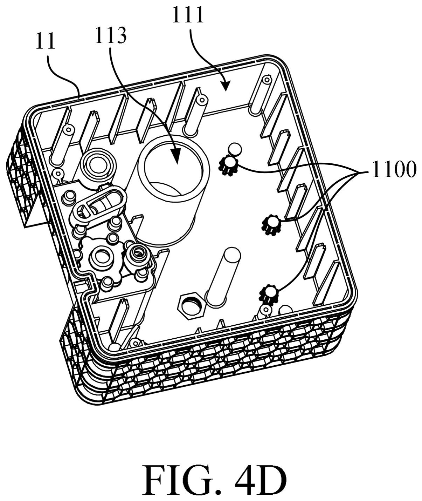

FIG. 4D is a schematic diagram illustrating the tank body of the water tank of FIG. 4A in another one perspective.

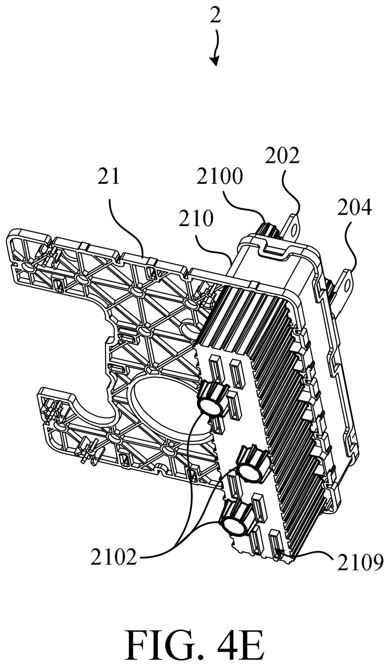

FIG. 4E is a schematic diagram illustrating the electrolysis module of FIG. 4A in another one perspective.

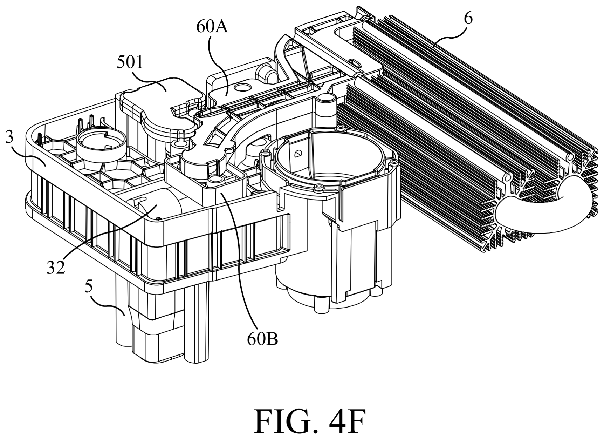

FIG. 4F is an assembly diagram illustrating the integrated channel device, the filter channel device and the condenser of FIG. 1.

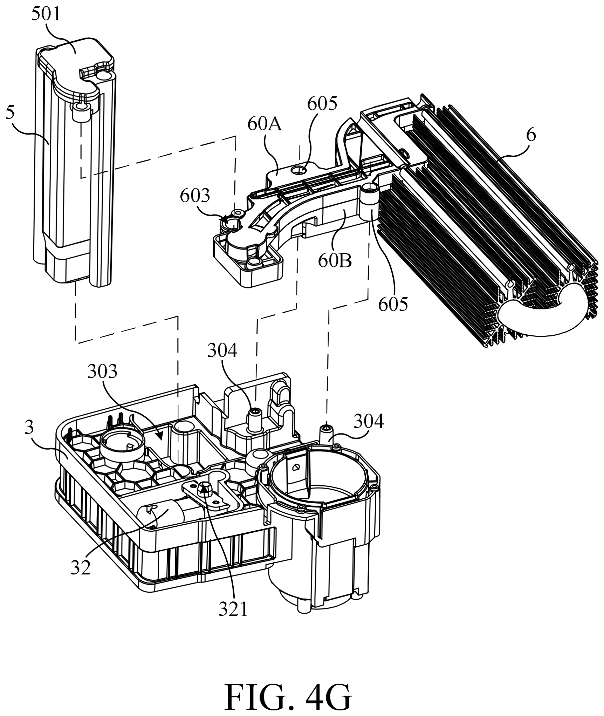

FIG. 4G is an exploded diagram illustrating the integrated channel device, the filter channel device and the condenser of FIG. 1.

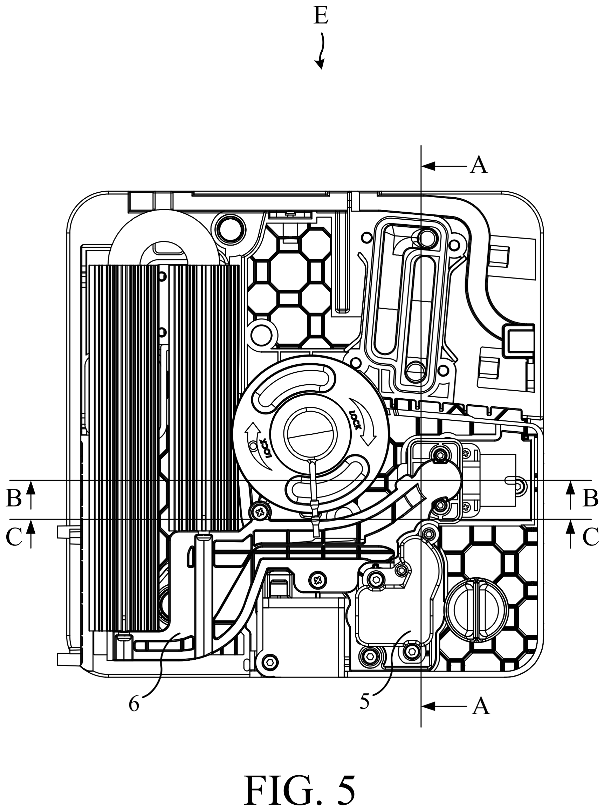

FIG. 5 is a structural schematic diagram illustrating the hydrogen generator with detachable filter of FIG. 1 in another one perspective.

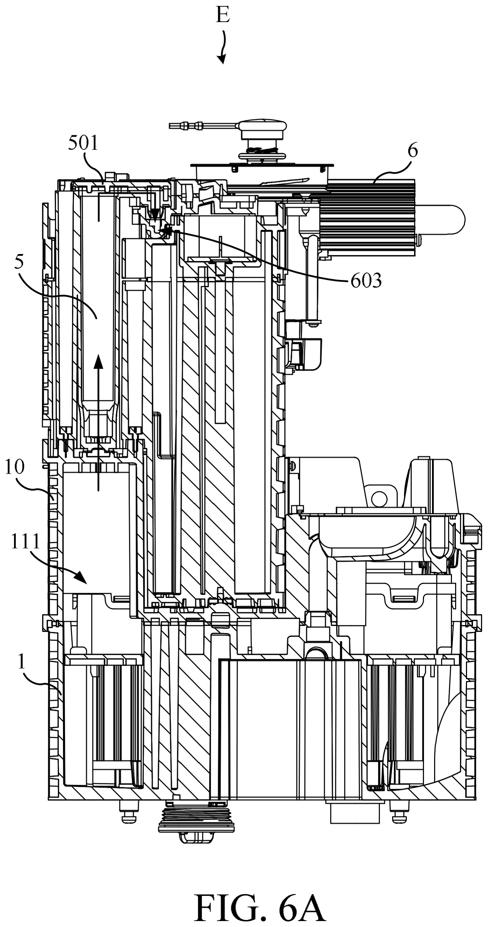

FIG. 6A is a sectional diagram illustrating the section A-A of FIG. 5.

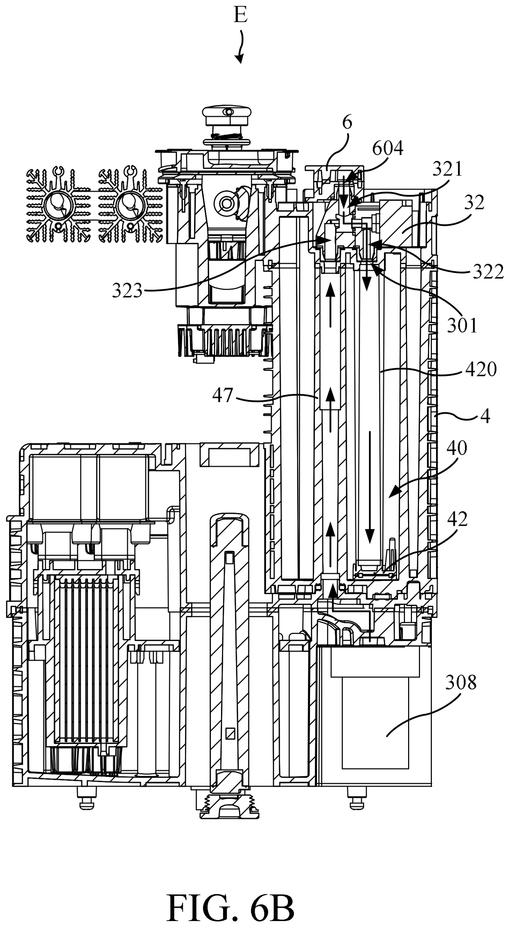

FIG. 6B is a sectional diagram illustrating the section B-B of FIG. 5.

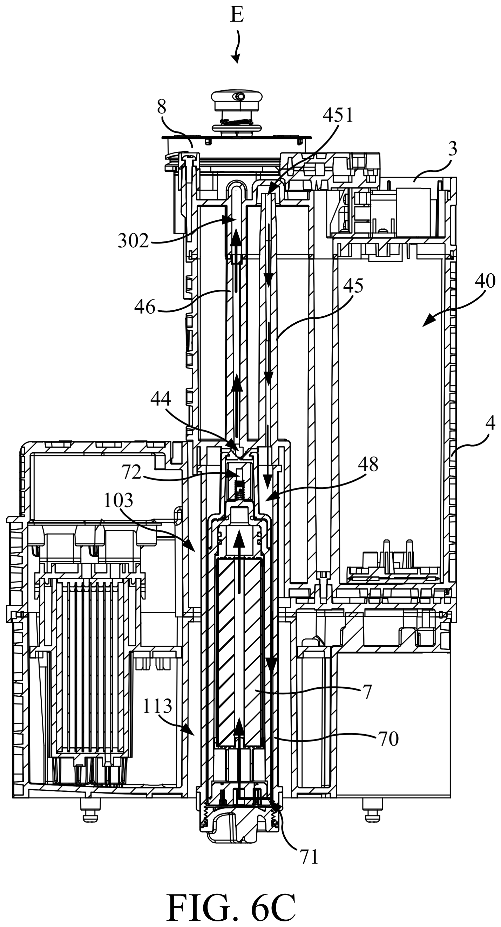

FIG. 6C is a sectional diagram illustrating the section C-C of FIG. 5.

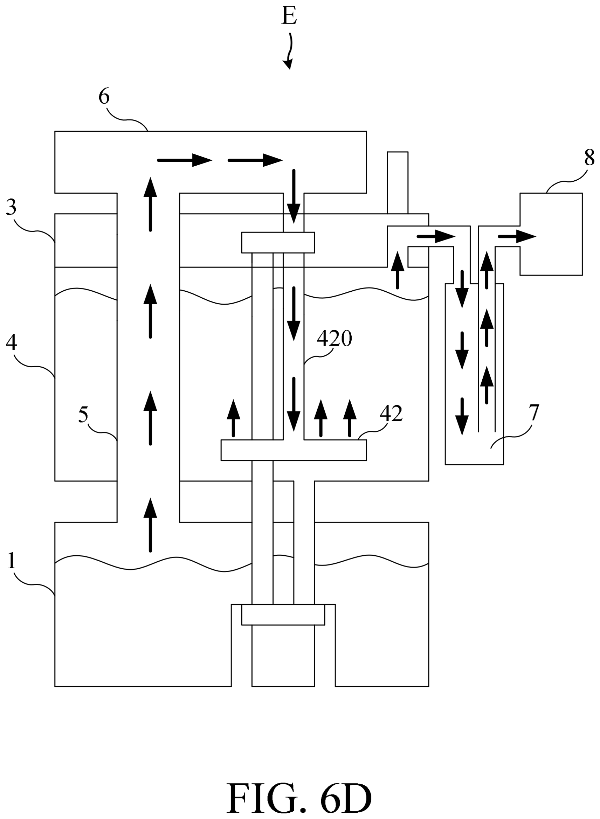

FIG. 6D is a simple schematic diagram illustrating the gas flow direction of the hydrogen generator with detachable filter according to an embodiment of the present invention.

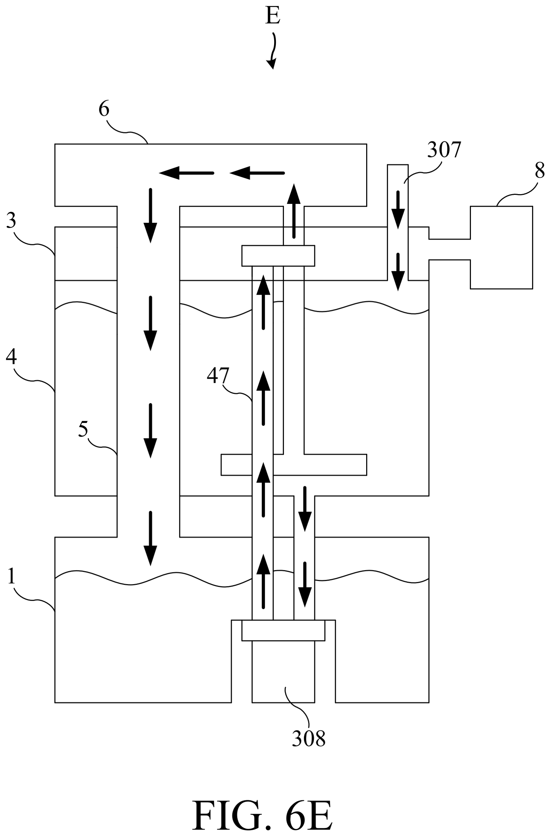

FIG. 6E is a simple schematic diagram illustrating the water replenish direction of the hydrogen generator with detachable filter according to an embodiment of the present invention.

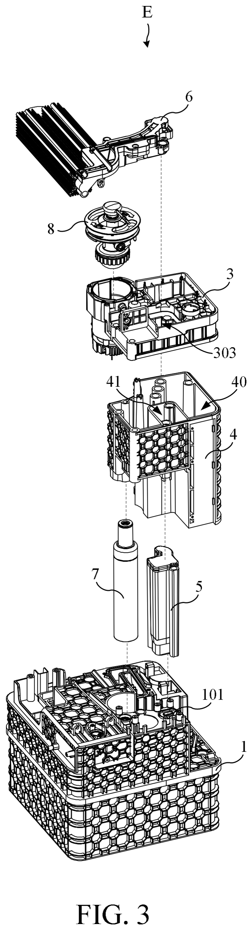

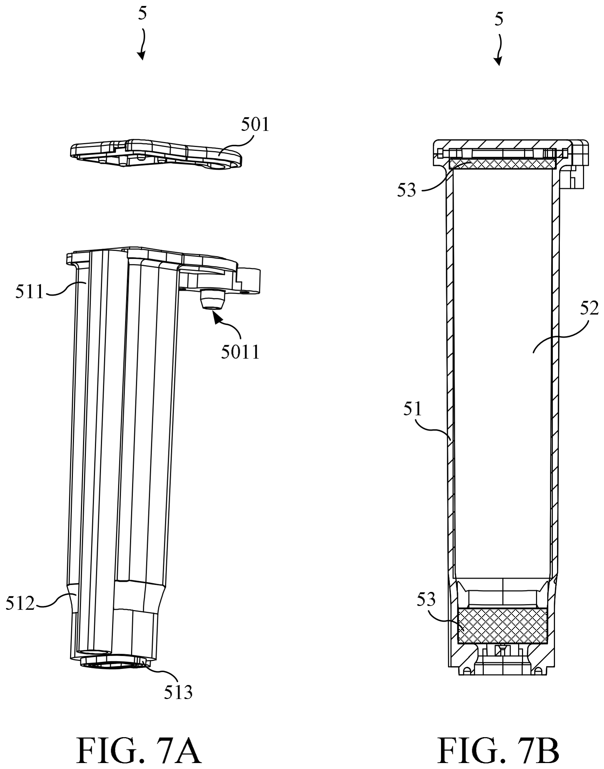

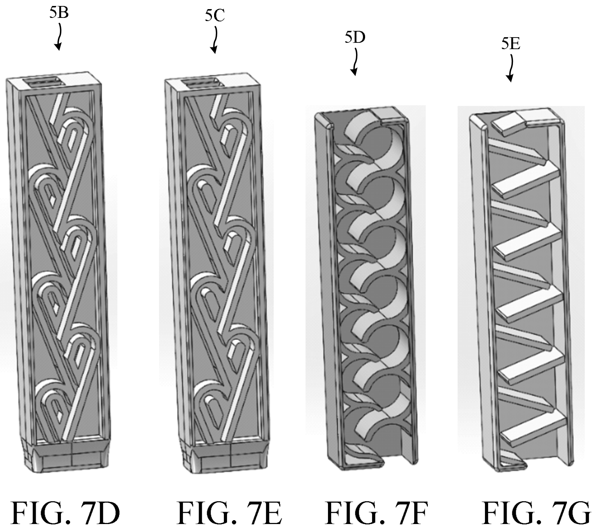

FIG. 7A is an exploded diagram illustrating the filter channel device of FIG. 3.
FIG. 7B is a sectional diagram illustrating the filter channel device of FIG. 3.

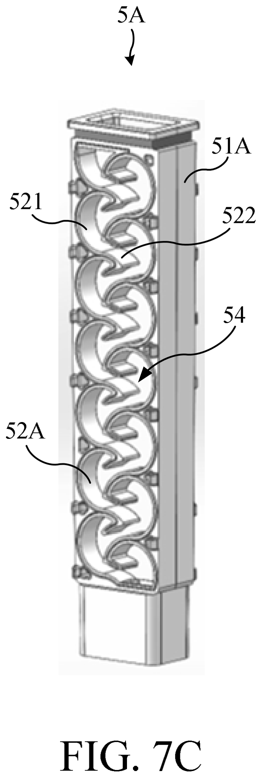

FIG. 7C to 7G are sectional diagrams illustrating the filter channel device of the hydrogen generator with detachable filter according to multiple embodiments of the present invention.

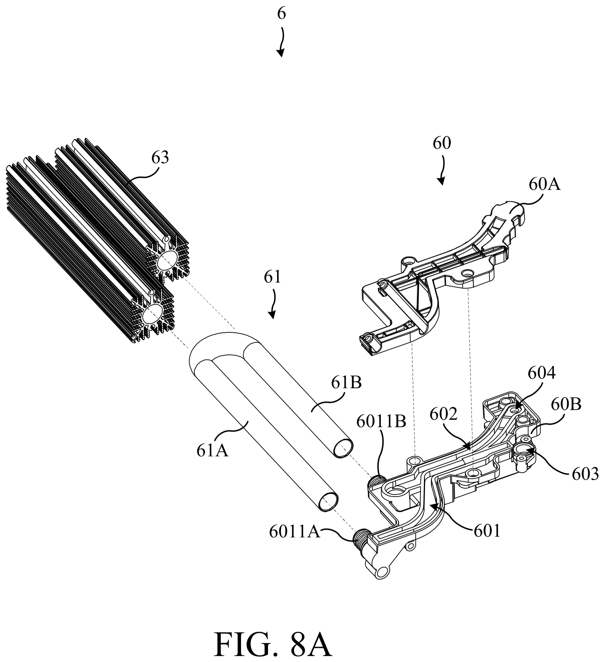

FIG. 8A is an exploded diagram illustrating the condenser of FIG. 3.

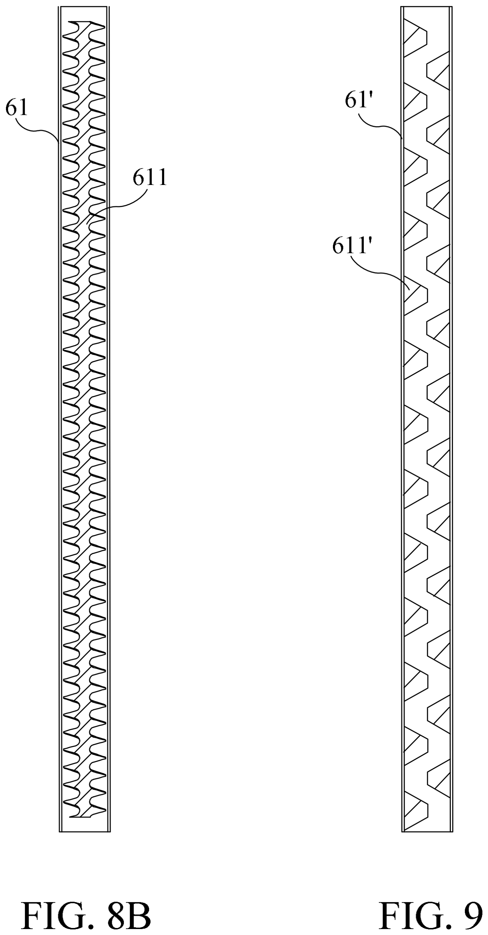

FIG. 8B is a sectional diagram illustrating the condenser tube of the condenser of FIG. 3.
FIG. 9 is a sectional diagram illustrating the condenser tube of the condenser according to another embodiment of the present invention.

The advantages and spirit of the present invention can be further understood by the following detailed description and with reference of the diagrams.

### DETAILED DESCRIPTION OF THE INVENTION

For the sake of the advantages, spirits and features of the present invention can be understood more easily and clearly, the detailed descriptions and discussions will be made later by way of the embodiments and with reference of the diagrams. It is worth noting that these embodiments are merely representative embodiments of the present invention, wherein the specific methods, devices, conditions, materials and the like are not limited to the embodiments of the present invention or corresponding embodiments. Moreover, the devices in the figures are only used to express their corresponding positions and are not drawing according to their actual proportion.

In the description of the present invention, it is to be understood that the orientations or positional relationships of the terms "longitudinal, lateral, upper, lower, front, rear, left, right, top, bottom, inner, outer" and the like are based on the orientation or positional relationship shown in the drawings. It is merely for the convenience of the description of the present invention and the description of the present invention, and is not intended to indicate or imply that the device or component referred to has a specific orientation, is constructed and operated in a specific orientation, and therefore cannot be understood as limitations of the invention.

In the description of this specification, the description with reference to the terms "a specific embodiment", "another specific embodiment" or "parts of specific embodiments" etc. means that the specific feature, structure, material or feature described in conjunction with the embodiment include in at least one embodiment of the present invention. In this specification, the schematic representations of the above-mentioned terms do not necessarily refer to the same embodiment. Moreover, the described specific features, structures, materials or characteristics can be combined in any one or more embodiments in a suitable manner.

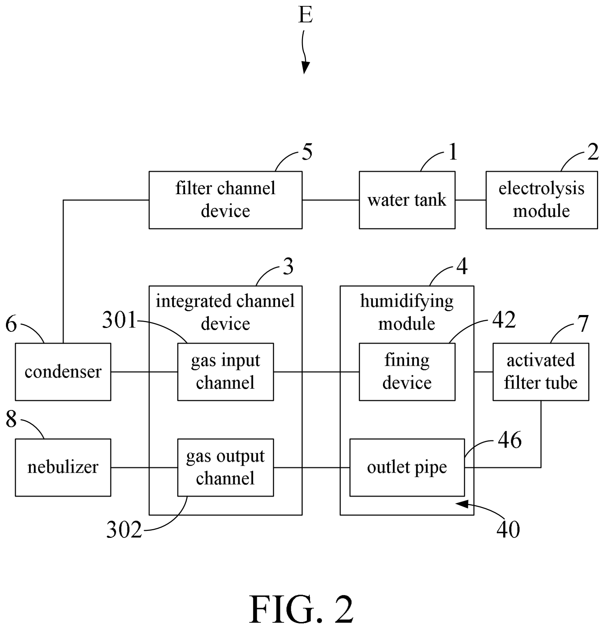

Please refer to FIG. 1 to FIG. 4B. FIG. 1 is a structural schematic diagram illustrating a hydrogen generator E with detachable filter according to an embodiment of the present invention. FIG. 2 is a functional block diagram illustrating the hydrogen generator E with detachable filter of FIG. 1. FIG. 3 is an exploded diagram illustrating the hydrogen generator E with detachable filter of FIG. 1. FIG. 4A is an exploded diagram illustrating the water tank of FIG. 1. FIG. 4B is a structural schematic diagram illustrating the humidifying module of FIG. 1. As shown in FIG. 1 to FIG. 4B, the hydrogen generator E with detachable filter comprises a water tank 1, an electrolysis module 2, an integrated channel device 3, a humidifying module 4, a filter channel device 5 and a condenser 6. The water tank 1 has an accommodation space 111 for accommodating an electrolyzed water. The electrolysis module 2 is arranged in the accommodation space 111 of the water tank 1, and the electrolysis module 2 is configured to receive the electrolyzed water from the water tank 1 and electrolyze the electrolyzed water to generate and output a gas comprising hydrogen in the water tank 1. The humidifying module 4 is stacked above the water tank 1 and configured to receive and humidify the gas comprising hydrogen. The humidifying module 4 has a humidifying chamber 40 and a gas channel 41. The humidifying chamber 40 and the gas channel 41 are isolated from each other. The filter channel device 5 penetrates the humidifying module 4 of the humidifying chamber 40 and is coupled with the water tank 1. The filter channel device 5 is configured to receive and filter the gas comprising hydrogen from the electrolysis module 2 and output the gas comprising hydrogen after filtering. The condenser 6 is arranged on the humidifying module 4 and fluidly coupled to the filter channel device 5. The condenser 6 is configured to receive and condense the gas comprising hydrogen from the filter channel device 5. The integrated channel device 3 is arranged on the humidifying module 4 and located between the humidifying module 4 and the condenser 6. The integrated channel device 3 is coupled to the condenser 6 and the humidifying module 4, and the integrated channel device 3 is configured to guide the gas comprising hydrogen from the condenser 6 into the humidifying module 4. The hydrogen generator with detachable filter E of the present invention is a stacked assembly structure, and it is arranged from bottom to top as follows: the water tank 1, the humidifying module 4, the integrated channel device 3 and the condenser 6.

In the present embodiment, the outer walls of the water tank 1 and the outer walls of the humidifying module 4 both comprise a plurality of ribs 13, and the plurality of ribs 13 form a honeycomb structure. In practice, the shape formed by the ribs 13 is not limited hereto. The plurality of ribs 13 are configured to strengthen the structural strength of the water tank 1 and the humidifying module 4 and to prevent the water tank 1 and the humidifying module 4 from deforming due to the different pressure caused by the generation and flow of the gas comprising hydrogen.

In the present embodiment, the water tank 1 includes a cover 10 and a tank body 11. The tank body 11 can form an accommodation space 111 to accommodate the electrolyzed water, and the cover 10 can cover the tank body 11 and accommodation space 111. The electrolysis module 2 is arranged in the accommodation space 111 of the water tank 1. Therefore, the electrolysis module 2 can be directly immersed in the accommodation space 111 with water and directly obtain water required for electrolysis from the accommodation space 111 without pipe connection. The electrolysis module 2 includes an electrolytic fixing plate 21, an electrolysis tank 210 and an electrode plate assembly component 20 arranged in the electrolysis tank 210. As shown in FIG. 2 simply, FIG. 2 only shows the part of the anode plate 202 and the part of the cathode plate 204 in the electrode plate assembly component 20. In the present embodiment, the electrolysis module 2 includes an anode plate 202, a cathode plate 204, and a bipolar electrode plate located between them. These electrode plates are arranged at interval in the electrolysis tank 210, so an electrode flow channel is formed between two electrode plates, and all electrode plates are formed multiple electrode flow channels, parallel to each other for electrolyzing water in them to generate hydrogen and oxygen, that is, gas comprising hydrogen. The top of the electrolysis tank 210 has a plurality of upper openings, respectively communicated with the electrode flow channel and the upper half part of the accommodation space 111. Correspondingly, the bottom of the electrolysis tank 210 has a plurality of lower openings (not shown in Fig. 4A), communicated with the electrode flow channel and the lower half part of the accommodation space 111. Through the upper openings and the lower openings, the electrolysis tank 210 can receive the electrolysis from the accommodation space 111 and electrolysis water in the electrode flow channel, and the gas comprising hydrogen is outputted from the electrode flow channel into the accommodation space 111. Therefore, the entire electrolysis module 2 of the hydrogen generator of the present invention is entirely located in the water tank, and it can dissipate heat through the water in the water tank at the same time. In practice, the hydrogen generator can include a cooling circulation system connected to the accommodation space of the water tank to cool and circulate the water in the accommodation space. At the same time, there is no need to carry water with any pipe between the electrolysis module and the water tank, so as to avoid water leakage or air leakage caused by pipe deterioration after long-term use.

The anode plate 202 and the cathode plate 204 can extend outward from the electrolysis tank 210. As shown in FIG. 4A, the extended portions of the anode plate 202 and the cathode plate 204 can be arranged in the holes of the cover 10 of the water tank 1, and contact the power source from the holes to receive the power required for electrolysis. Please refer to FIG. 4C. FIG. 4C is a structural schematic diagram illustrating the cover of FIG. 4A in another one perspective. As shown in FIG. 4C, The cover 10 has a through hole 1020 on one side, facing the accommodation space 111 for accommodating the anode plate 202 and the cathode plate 204. The cover 10 further has a first fixing part 1022 facing the accommodation space 111. The first fixing part 1022 includes two fixing rings, surrounding the circumference of the through hole 1020 shown in FIG. 4C respectively. In addition, the electrolysis tank 210 in FIG. 4A has a second fixing part 2108, and the second fixing part 2108 includes two rings, respectively surrounding the areas where the anode plate 202 and the cathode plate 204 extend. Therefore, when the electrolysis module 2 is arranged in the water tank 1, the anode plate 202 and cathode plate 204 can be located in the through hole 1020. Furthermore, the fixing ring of the first fixing part 1022 around the through hole 1020 and the anode plate 202 or the ring of the second fixing part 2108 around the cathode plate 204 are engaged with each other. Therefore, the electrolysis module 2 can be connected and linked to the cover 10 through the engagement between the first fixing part 1022 and the second fixing part 2108.

In practice, when the electrolysis module 2 electrolyzes water to generate the gas comprising hydrogen and the gas comprising hydrogen flows into the accommodation space 111 of the water tank 1, the gas comprising hydrogen will first accumulate at the top of the accommodation space 111 and output upward to the filter channel device 5. The pressure in the water tank 1 increases and leads the cover 10 to be pressed from the inside during gas comprising hydrogen accumulation. Since the electrolysis module 2 is suspended from the cover 10 and linked with each other, no matter how much the cover 10 bulges upward and deforms, the electrolysis module 2, the anode plate 202 and the cathode plate 204 maintain consistent relative positions with the cover 10 through contacting with the fixing ring of the first fixing part 1022 and the fixing ring of the second fixing part 2108. Similarly, when the electrolysis module 2 stops electrolysis or the water tank 1 relieves pressure inside, the cover 10 will return to its original shape or position, and the electrolysis module 2 will be linked to maintain a consistent relative position with the cover 10. Since the electrolysis module 2 can be linked with the cover 10 to maintain the relative position during change, there will not be any irreversible offset between the electrolysis module 2 and the cover 10 after long-term use, so as to reduce the situation of water leakage or air leakage. In particular, the parts of the anode plate 202 and the cathode plate 204 can be completely sealed in the through hole 1020 of the cover 10, so as to suppress the risk of steam entering the through hole 1020 to contact with the anode plate 202 and the cathode plate 204 .

In the present embodiment, the fixing ring of the first fixing part 1022 and the fixing ring of the second fixing part 2108 are jointed with each other through hot-melt manner, but it is not limited hereto, so that any fixing method can be firmly adopted in the present invention.

As shown in FIG 4C, the cover 10 has a first positioning structure 1024 on a surface facing the accommodation space 111, and the electrolysis tank 210 of the electrolysis module 2 has a second positioning structure 2100 corresponding to the first positioning structure 1024. The first positioning structure 1024 and the second positioning structure 2100 can be coupled to each other when the electrolysis module 2 is suspended from the cover 10, and can maintain the freedom of the first positioning structure 1024 and the second positioning structure 2100 to move up and down. In detail, the first positioning structure 1024 is a positioning hole, and the second positioning structure 2100 is a positioning post. When the electrolysis module 2 is suspended in the cover 10, the second positioning structure 2100 is located in the first positioning structure 1024 and can move up and down. Through the first positioning structure 1024 and the second positioning structure 2100, the lateral deflection between the cover 10 and the electrolysis module 2 can be suppressed when the cover 10 is deformed due to the accumulating pressure in the water tank 1, thereby further suppressing water leakage and air leakage. As mentioned above, in the present embodiment, two first positioning structures 1024 are both positioning holes, and two second positioning structures 2100 are both positioning posts. However, in practice, two first positioning structures 1024 can also be positioning posts, and two second positioning structures 2100 can also be positioning holes, or the first positioning structure and the second positioning structure may be a positioning post and a positioning hole respectively, which is not limited hereto. In addition, the first positioning structure 1024 and the second positioning structure 2100 are located on the side of the anode plate 202, the cathode plate 204 and the through hole 1020 of the cover 10, and the quantity is two. It should be noted that the quantity and location of the first positioning structure and the second positioning structure are not limited hereto; they can be determined according to the design or requirement.

On the other hand, please refer to FIG. 4D and FIG. 4E. FIG. 4D is a schematic diagram illustrating the tank body 11 of the water tank 1 of FIG. 4A in another one perspective. FIG. 4E is a schematic diagram illustrating the electrolysis module 2 of FIG. 4A in another one perspective. As shown in FIG. 4D and FIG. 4E, the tank body 11 of the water tank 1 has a third positioning structure 1100 at the bottom of the accommodation space 111, and the bottom of the electrolysis body forms a plurality of fourth positioning structure 2102, respectively corresponding to the third positioning structure 1100 of the tank body 11.

In the present embodiment, when the electrolysis module 2 is arranged in the accommodation space 111 of the water tank 1 and suspended from the cover 10, the third positioning structure 1100 of the tank body 11 of the water tank 1 is respectively coupled with the fourth positioning structure 2102 of the electrolysis body. In detail, the third positioning structure 1100 can be a positioning post, and the fourth positioning structure 2102 can be a tubular positioning structure, so the third positioning structure 1100 can be accommodated in the fourth positioning structure 2102. Moreover, since the third positioning structure 1100 and the fourth positioning structure 2102 extend vertically, the third positioning structure 1100 can move up and down in the fourth positioning structure 2102 without moving laterally. When the electrolysis module 2 electrolyzes water to produce the gas comprising hydrogen and the gas comprising hydrogen accumulates in the water tank 1, the cover 10 will be slightly bulged and deformed by the pressure of the gas comprising hydrogen in the accommodation space 111, so as to drive the electrolysis module 2 which is suspended from the cover 10 to move. When the electrolysis module 2 stops electrolyzing or the water tank 1 releases pressure, the pressure caused by the gas comprising hydrogen will be released in the water tank 1 and the cover 10 returns to its original state, which also drives the suspended electrolysis module 2 to move. Through the third positioning structure 1100 of the tank body 11 and the fourth positioning structure 2102 of the electrolysis tank 210, the direction of relative movement between the electrolysis module 2 and the tank body 11 can be limited and only move up and down. Since the electrolysis module 2 is suspended from the cover 10 and the electrolysis module 2 is not fixed in the tank body 11 through any screw, so the electrolysis module 2 may be tilted in the accommodation space 111 if the cover 10 is under pressure. Therefore, it is useful to prevent water leakage and air leakage by using the third positioning structure 1100 and the fourth positioning structure 2102, so as to let tank body 11 and the electrolysis module 2 only move up and down relatively to each other.

In the present embodiment, the quantity of the third positioning structure 1100 and the fourth positioning structure 2102 is three respectively, and they are respectively arranged at the positions shown in FIG. 4D and FIG. 4E. In practice, it should be noted that the quantity and the position of the third positioning structure and the fourth positioning structure are not limited in the present invention, and they can be determined according to the design or requirement. In order to effectively restrict the movement of the entire electrolysis module to only move up and down, the quantity of the third positioning structure and the fourth positioning structure can be at least two or more. The third positioning structure and the fourth positioning structure can be arranged at different locations such as on the bottom of the tank body and on the case of the electrolysis module, so as to prevent the electrolysis module from tipping sideways in the water tank. In addition, the first positioning structure 1024 of the cover 10 and the second positioning structure 2100 of the electrolysis tank 210 are coupled to each other as mentioned above. The first positioning structure 1024 and the second positioning structure 2100 can not only be determined the relative relationship between the cover and the electrolysis module 2, but also let the electrolysis module 2 move up and down along with the deformation or restoration of the cover 10.

In addition, as shown in FIG. 4E, the bottom of the electrolysis tank 210 has a plurality of lower openings 2109. As mentioned above, these lower openings 2109 communicate with the electrode flow channel formed by the plurality of electrodes in the electrolysis tank 210 and the lower half of part of the accommodation space 111. Since the electrolysis module 2 is disposed in the accommodation space 111 of the water tank 1, the electrolysis module 2 can directly receive the water in the water tank through lower opening 2109 for electrolysis to generate the gas comprising hydrogen. Therefore, the electrolysis module 2 of the hydrogen generator E can receive water without passing through the pipeline, so as to avoid water leakage or air leakage caused by pipe deterioration after long-term use.

In summary, the electrolysis module of the hydrogen generator of the present embodiment is suspended in the water tank, and is not only connected but also linked to the upper cover of the water tank. When the electrolysis module electrolyzes water to generate the gas comprising hydrogen, the pressure in the water tank will increase. Furthermore, the cover of the water tank will be bulged deformation caused by the pressure increasing. However, the electrolysis module can maintain a relative position to the cover when the water tank moves. Therefore, the electrode plates provided in the electrolysis module can still be enclosed in the cover, and reduces the risk of water leak and air leak after long-term use. In addition, the positioning structures on the tank body, the cover and the case of the electrolysis module can prevent the electrolysis module from lateral deviation or tilt in the water tank, so as to avoid the risk of leakage

In addition, the electrolytic fixing plate 21 of the electrolysis module 2 further includes a partition plate 211. The partition plate 211 is configured to fix the electrolysis module 2 in the water tank 1, and can divide the water tank 1 into upper layer and lower layer. Then, the electrolyzed water is mainly located in the lower layer, and the gas comprising hydrogen generated by electrolysis is mainly located in the upper layer. In order to maintain circulation between the upper layer and lower layer, the partition plate 211 has a plurality of circulation holes 2110 to connect the upper layer and lower layer. The electrolytic fixing plate 21 can be an integrally formed structure. In addition, it should be noted that the shape of the electrolytic fixing plate 21 can be determined according to the design or requirement of the skilled in the art, so as to provide space for accommodating other components.

Please refer to FIG. 3, FIG. 4F, FIG. 4G, FIG. 5 and FIG. 6B. FIG. 4F is an assembly diagram illustrating the integrated channel device 3, the filter channel device 5 and the condenser 6 of FIG. 1. FIG. 4G is an exploded diagram illustrating the integrated channel device 3, the filter channel device 5 and the condenser 6 of FIG. 1. FIG. 5 is a structural schematic diagram illustrating the hydrogen generator E with detachable filter of FIG. 1 in another one perspective. FIG. 6B is a sectional diagram illustrating the section B-B of FIG. 5. As shown in FIG. 3, FIG. 4F, FIG. 4G, in the present embodiment, the humidifying module 4 is stacked vertically above the water tank 1; the integrated channel device 3 is stacked vertically above the humidifying module 4; the condenser 6 is fixed above the integrated channel device 3; and the filter flow channel device 5 penetrates through the humidifier 4 and the integrated flow channel device 3 to directly connect the condenser 6. The gas channel 41 of the humidifying module 4 is a through hole extending vertically upward from the bottom of the humidifying module 4 to the top. Furthermore, the integrated channel device 3 includes an opening 303 corresponding to the gas channel 41 of the humidifying module 4. The bottom of the filter channel device 5 can pass through the opening 303 of the integrated channel device 3 and the gas channel 41 of the humidifying module 4 to connect the water tank 1. Therefore, the gas comprising hydrogen generated in the water tank 1 can directly flow to the filter channel device 5 and the condenser 6 in sequence, without flowing to the inside of the integrated channel device 3 and the inside of the humidifying module 4. In addition, the length of the filter channel device 5 is larger than the length of the hole 303 combined with the gas channel. When the hydrogen generator E with detachable filter is assembled, the top of the filter channel device 5 protrudes from the integrated channel device 3. Therefore, if the user wants to replace or clean the filter flow channel device 5, the user can hold the top of the filter channel device 5 and then directly pull out the filter channel device 5 from the integrated channel device 3 without disassembling other components or devices, so as to improve convenience and installation efficiency.

As shown in FIG. 4G, in the present embodiment, the integrated channel device 3 includes a fixing structure 304, and the condenser 6 includes a matching structure 605 matching and corresponding to the fixing structure 304. The matching structure 605 of the condenser 6 can be connected to the fixing structure 304 with any locking method to fix the condenser 6 on the integrated channel device 3. Since the condenser 6 is located at the top of the entire the hydrogen generator E, and the filter channel device 5 is an extractable and detachable component. Therefore, the user can directly disassemble and clean the condenser 6 after extracting the filter channel device 5 without disassembling other components or devices, so as to improve convenience and installation efficiency.

Please refer to FIG. 1, FIG. 3, FIG. 7A, and FIG. 7B. FIG. 7A is an exploded diagram illustrating the filter channel device 5 of FIG. 3. FIG. 7B is a sectional diagram illustrating the filter channel device 5 of FIG. 3. As shown in FIG. 3, FIG. 7A, and FIG. 7B, in the present embodiment, the filter channel device 5 includes a flow channel housing 51, and the size of the flow channel housing 51 can correspond to the size of the gas channel 41 of the humidifying module 4 and the opening 303 of the integrated channel device 3. The flow channel housing 51 has a first end 511 and a second end 512, and there is an upper cover 501 arranged on the first end 511. The first end 511 protrudes from the integrated channel device 3 and is directly connected to the condenser 6. The second end 512 is connected to cover 10 of the water tank 1. The second end 512 of the flow channel housing 51 further includes an installing structure 513, and the cover 10 of the water tank 1 includes an assembly structure 101 matching the installing structure 513. When the hydrogen generator E with detachable filter is assembled, the installing structure 513 of the flow channel housing 51 can be engaged and tightly fitted on the assembly structure 101 of the water tank 1. Therefore, the gas comprising hydrogen generated from the electrolysis module 2 can directly flow from the water tank 1 to the filter channel device 5, so as to prevent air leakage from the inside of the water tank 1.

Further, the filter channel device 5 has an opening 5011, and the installing structure 513 and the assembly structure 101 of the water tank 1 has openings respectively. That is to say, the opening 5011 of the filter channel device 5, the cavity of the flow channel housing 51, the opening of the installing structure 513, the opening of the assembly structure 101 and the accommodation space 111 of the water tank 1 are connected with each other. Therefore, when the electrolysis module 2 of the hydrogen generator E with detachable filter of the present invention electrolyzes the electrolyzed water and generates the gas comprising hydrogen, the gas comprising hydrogen flows through the accommodation space 111 of the water tank 1, the opening of the installing structure 513 and then flows to the filter channel device 5. Next, after the filter channel device 5 filters the gas comprising hydrogen, and then the gas comprising hydrogen flows through the opening 5011 to the condenser 6.

In the present embodiment, the filter channel device 5 further includes a filter component 52 arranged in the cavity of the flow channel housing 51 for filtering alkali, impurities and electrolytes in the gas comprising hydrogen generated from the electrolysis module 2. In practice, the filter component 52 can be filter cotton, but it is not limited hereto. As shown in FIG. 7B, the filter channel device 5 further includes two metal mesh components 53 respectively arranged and fixed on the first end 511 and the second end 512, and the filter component 52 is located between two metal mesh components 53. In practice, when the gas comprising hydrogen generated from the electrolysis module 2 flows through the water tank 1 to the filter channel device 5, the filter component 52 located in the flow channel housing 51 may be moved by flowing through the gas comprising hydrogen. Therefore, the metal mesh component 53 restricts the movement range of the filter component 52. In addition, the metal mesh component 53 has the structure with gaps, so the gas comprising hydrogen can flow through the metal mesh component 53 without being blocked. Furthermore, the metal mesh component 53 also has a filtering function to filter alkali, impurities and electrolytes in the gas comprising hydrogen. In another specific embodiment, the filter channel device can only include metal mesh component without filter cotton. Furthermore, the metal mesh element 53 may also have a filtering function to filter alkalis, impurities and electrolytes in the gas comprising hydrogen.

The hydrogen generator E with detachable filter of the present invention further includes a conductive component (such as a metal rod). The conductive component can extend downward from the bottom of the filter channel device 5 to the electrolyzed water in the water tank 1. In one present embodiment, one end of the conductive component is arranged at the bottom of the filter channel device 5, and the other end of the conductive component extends into the water tank 1.

Please refer to FIG. 3, FIG.5, FIG.8A, and FIG.8B. FIG. 8A is an exploded diagram illustrating the condenser 6 of FIG. 3. FIG. 8B is a sectional diagram illustrating the condenser tube 61 of the condenser 6 of FIG. 3. As shown in FIG. 8A, in the present embodiment, the condenser 6 includes a base 60 and a condensation tube 61. The base 60 includes an upper base 60A and a lower base 60B that can match with each other. The lower base 60B includes a first flow channel 601 and a second flow channel 602 that are isolated from each other. Further, the lower base 60B has a condensation inlet 603 and a condensation outlet 604. The condensation inlet 603 is connected to the first flow channel 601 and directly connected to the upper cover 501 of the filter channel device 5 (as shown in FIG. 4G and FIG. 7A), and the condensation outlet 604 is connected to a gas input channel 301 of the integrated channel device 3 (as shown in FIG. 6B). In practice, the upper base 60A can also include two flow channels that respectively correspond to and match the first flow channel 601 and the second flow channel 602, so that the upper base 60A of the condenser 6 forms two isolated and separated flow channels. However, the shape of the upper base is not limited hereto. The upper base can also be a flat plate without a flow channel. The gas comprising hydrogen flows only in the first flow channel 601 and the second flow channel 602 of the lower base.

As shown in FIG.3 and FIG.5, in the present embodiment, the shape of the condenser 6 is approximately L-shaped. When the condenser 6 is assembled, the base 60 of the condenser 6 is installed on the top surface of integrated channel device 3, and the condensation tube 61 of the condenser 6 is suspended from the side of the integrated channel device 3. Furthermore, the length of the base 60 of the condenser 6 accounts for more than two-thirds of the side length of the hydrogen generator E. Therefore, when the matching structure 605 of the condenser 6 is locked to the fixing structure 304 of the integrated channel device 3 (as shown in FIG. 4G), the condenser 6 can be more firmly fixed on the integrated channel device 3 and support the condensation tube 61. In practice, the length of the base 60 of the condenser 6 is not limited hereto, and the length of the base 60 can account for more than half of the side length of the hydrogen generator E.

In practice, the condensation tube 61 of the condenser 6 includes a first condensation tube 61A and a second condensation tube 61B, and the lower base 60 has a first connection hole 6011A and a second connection hole 6011B with threads. One end of the first condensation tube 61A is connected to the first connection hole 6011A to communicate with the first flow channel 601, and one end of the second condensation tube 61B is connected to the second connection hole 6011B to communicate with the second flow channel 602. A through connecting tube is connected to one end of the first condensation tube 61A and one end of the second condensation tube 61B, which forms a U-shape tube. Therefore, the condensation tube 61 is a single channel. The other ends of the first condensation tube 61A and the second condensation tube 61B include internal threads that respectively match the first connection hole 6011A and a second connection hole 6011B. When the condenser 6 is assembled, the condensation inlet 603, the first flow channel 601, the first condensation tube 61A, the second condensation tube 61B, the second flow channel 602 and the condensation outlet 604 form a single path condensation flow channel. In the present embodiment, the first condensation tube 61A and the second condensation tube 61B are arranged horizontally side by side to reduce the volume and height of the hydrogen generator E with detachable filter. In practice, the arrangement of the first condensation tube and the second condensation tube can be determined according to the design or requirement. Furthermore, the condenser 6 can also include more than two condensation tubes, and multiple connecting tubes are connected to the connect condensation tubes to form a single channel.

In addition, the condenser 6 further includes a heat dissipation element 63, and the heat dissipation element 63 is contacted to and arranged outside of the first condensation tube 61A and the second condensation tube 61B. In the present embodiment, the heat dissipation element 63 is an aluminum extrusion, and the heat dissipation element 63 has holes corresponding to the size of the condensation tube, and the holes is configured to make the condensation tube passes through and dissipate heat. In practice, aluminum is a good thermal conductivity material. When the gas comprising hydrogen flows through the condensation tube 61, the aluminum extrusion absorbs the heat energy from the gas comprising hydrogen and conducts heat exchange with air from outside, and the steam is condensed into water, so as to improve the condensation efficiency.

As shown in FIG. 8B, the condenser 6 further includes a plurality of spiral structure 611 respectively arranged in the first condensation tube 61A and the second condensation tube 61B of the condensation tube 61 (FIG. 8B only illustrates one of the second condensation tube 61B). In practice, the spiral structure 611 can be an I-shaped spiral column arranged in the condensation tube 61 to extend the path length of the first condensation tube 61A and the second condensation tube 61B. That is to say, the spiral structure 611 is configured to increase the length of the condensation channel. Therefore, when the gas comprising hydrogen flows through the condensation tube 61 of the condenser 6, the gas comprising hydrogen flows through the condensation tube 61 along the spiral structure 611,so as to increase the time staying in the condensation tube 61 and improve the condensation efficiency.

Please refer to FIG. 4B-1, FIG. 4B-2, FIG.4G and FIG.6B. FIG. 4B-1 is a structural schematic diagram illustrating the humidifying module 4 of FIG. 1 in another one perspective. FIG. 4B-2 is a structural schematic diagram illustrating the fining structure 42. In the present embodiment, the hydrogen generator E with detachable filter includes a valve 32 arranged on the integrated channel device 3 and connected to the condenser 6 and the humidifying module 4. The integrated channel device 3 includes the gas input channel 301. The valve 32 includes a first valve interface 321 and a second valve interface 322. The first valve interface 321 is connected to the condensation outlet 604 of the condenser 6, and the second valve interface 322 is connected to the gas input channel 301 of the integrated channel device 3. Furthermore, the hydrogen generator E with detachable filter further includes a fining device 42 arranged in the humidifying chamber 40 of the humidifying module 4. The fining device 42 includes a communication pipe 420 connected to the gas input channel 301 of the integrated channel device 3 to input the gas comprising hydrogen from the condenser 6 into the replenishing water of the humidifying chamber 40. In practice, part of the fining device 42 can be arranged in the replenishing water. As shown in FIG. 6B, the fining device 42 is arranged at the bottom of the humidifying module 4, but it is not limited hereto. Further, the fining device 42 can include a plurality of micro holes 421. When the condenser 6 outputs the gas comprising hydrogen after being condensed, the gas comprising hydrogen flows through the condensation outlet 604, the first valve interface 321, the second valve interface 322, the gas input channel 301 and the communication pipe 420 of the fining device 42. Next, the gas comprising hydrogen flows through the micro holes 421 of the fining device 42 and enters into the replenishing water of the humidifying chamber 40 to form micro bubbles, and the gas comprising hydrogen is fully filtered and humidified by the supplementary water in the humidifying chamber 40.

Furthermore, the electric potential of the water tank 1, the humidifying module 4, the filter channel device 5, the condenser 6 and the integrated channel device 3 are equal to that of each other. The hydrogen generator E with detachable filter further includes a case (not shown) for accommodating the components as above. The water tank 1, the humidifying module 4, the filter channel device 5, the condenser 6 and the integrated channel device 3 and other components can be electrically connected to the case.

Please refer to FIG. 3, FIG. 4C, FIG. 4D, and FIG. 6C. FIG. 6C is a sectional diagram illustrating the section C-C of FIG. 5. In the present embodiment, the hydrogen generator E with detachable filter further includes an activated carbon tube or an activated filter tube 7 fluidly connected to the humidifying module 4 and the integrated channel device 3. As shown in FIG. 3, FIG. 4C, FIG. 4D, and FIG. 6C, the cover 10 of the water tank 1 includes the cover channel 103, and the tank body 11 includes the tank channel 113. Wherein, the cover channel 103 and the tank channel 113 correspond to each other, but the inside space of the cover channel 103 and the internal space of the cover 10 are isolated from each other. Moreover, the tank channel 113 and the assembly structure 101 are isolated from each other. The bottom of the humidifying module 4 has an inlet pipe 45 and an outlet pipe 46. The installation interface 44, the outlet pipe 46, the cover channel 103, and the tank channel 113 are correspond to and connected with each other. The inlet pipe 45 connects to the humidifying chamber 40 and includes an inlet intake 451, and the inlet pipe 45 does not connect the outlet pipe 46. The activated filter tube 7 includes a shell 70, and the top of the activated filter tube 7 can penetrate the tank channel 113 and the cover channel 103, and further engages with the installation interface 44 to connect with the humidifying module 4. The active filter tube 7 includes a shell 70, and the top of the active filter tube 7 can penetrate the box channel 113 and the cover channel 103 and engage with the installation interface 44 to connect with the humidifier 4. Further, the inlet pipe 45 is higher than the water surface of the replenishing water in the humidifying chamber 40. When the active filter tube 7 is installed on the humidifier 4, the top of the shell 70 is close to the bottom of the humidifying module 4. At this time, a cavity 48 is formed between the filter tube 7 of the activated filter tube 7 and the bottom of the humidifier 4, and the humidifying chamber 40, the inlet intake 451 of the inlet pipe 45 and the cavity 48 are connected with each other. In addition, the active filter tube 7 is configured to filter the gas comprising hydrogen in the humidifying module 4, and the bottom of the active filter tube 7 includes an air inlet 71 and an air outlet 72. The air inlet 71 is connected to the cavity 48, and the air outlet 72 is connected to the installation interface 44 and the outlet pipe 46. When the humidifying module 4 receives and humidifies the gas comprising hydrogen after condensing from the condenser 6, the humidified gas comprising hydrogen flows from the inlet intake 451 of the inlet pipe 45 to the cavity 48, and further flows through the air inlet 71 to the activated filter tube 7, so as to filter impurities. Furthermore, the integrated channel device 3 further includes an outlet flow channel 302 connected to the outlet pipe 46 of the humidifying chamber 40. Therefore, the gas comprising hydrogen filtered by the activated filter tube 7 flows sequentially from the air outlet 72 through the installation interface 44, the outlet pipe 46 and the outlet flow channel 302 to output the gas comprising hydrogen from the humidifying module 4.

In addition, the hydrogen generator E with detachable filter further includes nebulizer 8 coupled to the outlet flow channel 302 of the integrated channel device 3 to receive the gas comprising hydrogen, and can selectively generate an atomized gas and the atomized gas can mix with the gas comprising hydrogen to form a health-care gas. The nebulizer 8 can generate the atomized gas health-care gas mixed with the atomized gas and the gas comprising hydrogen. Wherein, the atomized gas can select such as steam, atomized medicine or essential oils to mix with. In one present embodiment, the nebulizer 8 includes an oscillator to nebulizer the water, atomized medicine or essential oils in the nebulizer 8 to generate the atomized gas, and then mixes with the mixing gas to from the health-care gas. Further, the nebulizer 8 can be selectively turned on or turned off according to the user's requirement to provide health-care gas mixed with the atomized gas for the user to inhale, or only provide the mixed gas (the hydrogen gas diluted with the oxygen) for the user to inhale.

Please refer to FIG. 3, and FIG. 6A to FIG. 6D. FIG. 6D is a simple schematic diagram illustrating the gas flow direction of the hydrogen generator E with detachable filter according to an embodiment of the present invention. The gas flow direction of the gas comprising hydrogen is shown by the arrow in FIG. 6D. When the hydrogen generator E with detachable filter of the present invention is operating, first, the electrolysis module 2 in the water tank 1 electrolyzes the electrolyzed water to generate the gas comprising hydrogen, and then the gas comprising hydrogen flows from the accommodation space 111 of the water tank 1 through the filter channel device 5 and the opening 5011 of the filter channel device 5, and then through the condensation inlet 603 to the condenser 6. It should be noted that while the condensation tube 61 condenses the steam in the gas comprising hydrogen, the condensed water will take away the remaining electrolyte in the gas comprising hydrogen. Therefore, the condenser 6 also contains other function for filtering. Next, the gas comprising hydrogen sequentially flows through the condensation outlet 604 of the condenser 6, the gas input channel 301 of the integrated channel device 3, the micro holes of the fining device 42 of the humidifying module 4 and enters into the humidifying chamber 40 of the humidifying module 4 to humidify the gas comprising hydrogen. Then, the gas comprising hydrogen flows from the humidifying module 4 through the outlet flow channel 302 of the integrated channel device 3, the activated filter tube 7 and enters into the nebulizer 8. Finally, the atomizer 8 can be selectively opened or closed according to the user's needs to provide the health-care gas mixed with the atomized gas for the user to inhale.

Since the hydrogen generator generates the gas comprising hydrogen, the amount of water in the device will gradually decrease, so it is necessary to regularly replenish water in the water tank for a period of time. Please refer to FIG. 4B, FIG. 6B and FIG. 6E. FIG. 6E is a simple schematic diagram illustrating the water replenish direction of the hydrogen generator E with detachable filter according to an embodiment of the present invention. As shown in FIG. 4B, FIG. 6B, the humidifying module 4 further includes a reflow pipe 47, the valve 32 includes a third valve interface 323, and the reflow pipe 47 is connected to the third valve interface 323. The hydrogen generator E with detachable filter further includes a water replenishing pump 308 arranged on the outside of the water tank 1 and connected to the reflow pipe 47. The water replenish direction is shown by the arrow in FIG. 6. When the hydrogen generator E with detachable filter of the present invention replenishes water, the replenishing water can be replenished into the humidifying chamber 40 of the humidifying module 4 from the water replenishing inlet 307 of the humidifying module 4. Next, the water replenishing pump 308, arranged on the outside of the water tank 1 can directly transported the replenishing water in the humidifying chamber 40 to the condenser 6. Finally, the replenishing water flows from the condenser 6 through the filter channel device 5 and back into the water tank 1. It should be noted that when the replenishing water flows from the condenser 6 to the water tank 1, the replenishing water will also carry alkali and electrolyte remaining on the condenser 6 and the filter channel device 5 back to the water tank 1. The first valve interface 321 of the valve 32 can selectively communicate with the second valve interface 322 or the third valve interface 323 according to the operating status. For example, when the hydrogen generator E with detachable filter of the present invention operates, the first valve interface 321 of the valve 32 is connected to the second valve interface 322, and the gas comprising hydrogen after being condensed in the condenser 6 flows into the fining device 42 for humidification. For another example, when the hydrogen generator E replenishes water, the first valve interface 321 of the valve 32 is the third valve interface 323, and the replenishing water flows through the reflow pipe 47 and the condenser 6 and back into the water tank 1.

In addition, the filter channel device of the hydrogen generator with detachable filter of the present invention can be in the specific embodiments as described, the filter channel device may also adopt other structures or configurations. Please refer to FIG. 7C to FIG. 7G. FIG. 7C to 7G are sectional diagrams illustrating the filter channel device of the hydrogen generator with detachable filter according to multiple embodiments of the present invention. As shown in FIG. 7C, the difference between the filter channel device 5A in this specific embodiment and the previous specific embodiment is that the filter component 52 of the filter channel device 5A is a plurality of baffle structures, and each baffle structure includes an arc part 521 and a bottom hook part 522 connecting the arc part 521. Wherein, the arc part 521 extends upward, and the bottom hook part 522 extends downward from the top of the arc part 521. Furthermore, the baffle structures are staggered and configured in the flow channel housing 51 to form a filter channel. In practice, the arc part 521 can extends upward along the inner wall of the filter channel device 5A, and the bottom hook part 522 can extend from the top of the arc part 521 toward the lower right direction, and extend toward the lower left direction. The baffle structures can stagger and protrude from two opposite inner walls of the flow channel housing 51A toward the cavity of the flow channel housing 51A, and the baffle structures can be integrally formed with the flow channel housing 51A. Furthermore, the filter channel can be a S-shaped flow channel. When the gas comprising hydrogen generated from the water tank 1 flows through the filter channel 54 of the filter channel device 5A, the bottom hook part 522 of the baffle structures will block alkali, impurities and electrolytes in the gas comprising hydrogen to keep from passing through, which means the alkali, impurities and electrolytes will be attached on the baffle structures of the filter channel device 5A, so as to achieve the filtration effect. Similarly, when the hydrogen generator E with detachable filter replenishes water, the replenishing water will flow through the filter channel 54 of the filter channel device 5A. Furthermore, the replenishing water will flush the alkali, impurities and electrolytes on the baffle structures back to the water tank 1 at the same time. It should be noted that the shapes of the baffle structures are not limited to those described in FIG. 7C, the baffle structures can also be other statuses shown in filter channel device 5B, 5C, 5D and 5E in FIG. 7D to 7G. In practice, the bottom hook part 522 of the baffle structure can also only extend toward the lower right or lower left direction.

The condenser of the hydrogen generator with detachable filter of the present invention can be in the specific embodiments as described, the condenser can also be in other statuses. Please refer to FIG. 9. FIG. 9 is a sectional diagram illustrating the condenser tube 61 of the condenser according to another embodiment of the present invention. As shown in FIG. 9 in the present embodiment, the inner wall of the condensation tube 61' has a delay structure 611'. In practice, the inner surface of the condensation tube 61' has a plurality of bumps to form the delay structure 611' to increase the path length of the condensation flow channel. Furthermore, the bumps can also be formed as internal thread structures on the inner surface of the condensation tube 61'. In another present embodiment, the delay structure 611' are not limited to bumps on the surface, and the delay structure 611' can further include other structures to delay the flow rate of liquid, such as mesh structures. In addition, in another present embodiment, the heat dissipation element of the condenser is a plurality of heat dissipation fins. The heat dissipation fins has a plurality of holes, and the condensation tube can be arranged in the holes. In practice, the heat dissipation fins can be a two-piece combined structure or a three-dimensional wavy structure to increase the heat dissipation surface area. Moreover, the heat dissipation fins can be in other statuses, such as without holes on the heat dissipation fins. The heat dissipation fins can surround the condenser tube to dissipate heat. Furthermore, the plurality of heat dissipation fins can be arranged at fixed intervals.

In summary, the hydrogen generator with detachable filter of the present invention has a separate filter channel device and a condenser. The user can directly pull out the filter channel device and disassemble the condenser without disassembling other components or devices when the filter channel device and the condenser need to be cleaned or the filter channel device needs to be replaced, so as to increase convenience and installation efficiency. In addition, the condenser of the hydrogen generator with detachable filter of the present invention can effectively improve the condensation path and heat dissipation function through the extendable condensation flow channel and the heat dissipation element so as to improve condensation and filtration efficiency.

With the examples and explanations mentioned above, the features and spirits of the invention are hopefully well described. More importantly, the present invention is not limited to the embodiment described herein. Those skilled in the art will readily observe that numerous modifications and alterations of the device may be made while retaining the teachings of the invention. Accordingly, the above disclosure should be construed as limited only by the metes and bounds of the appended claims.

## Claims

1. A hydrogen generator with detachable filter, comprising:
a water tank having an accommodation space for accommodating an electrolyzed water;
an electrolysis module configured in the accommodation space of the water tank, the electrolysis module being configured to receive and electrolyze the electrolyzed water from the water tank to generate and output a gas comprising hydrogen;
a humidifying module stacked above the water tank to humidify the gas comprising hydrogen, the humidifying module having a humidifying chamber for accommodating a replenishing water;
a filter channel device coupled with the water tank, the filter channel device comprising a channel case and a filter component in the channel case, the filter channel device being configured to receive and filter the gas comprising hydrogen from the electrolysis module and output the gas comprising hydrogen after filtering;
a condenser stacked above the water tank, the condenser being fluidly coupled to the filter channel device to receive and condense the gas comprising hydrogen from the filter channel device; and
an integrated channel device stacked above the water tank, the integrated channel device comprising a gas input channel fluidly coupling the condenser and the humidifying chamber to guide the gas comprising hydrogen from the condenser into the humidifying chamber;
wherein, the filter channel device, the condenser and the humidifying device are respectively engaged with the integrated channel module; the filter channel device penetrates the humidifying module and the integrated channel module, and the filter channel device is detachable from the humidifying module and the integrated channel device.

2. The hydrogen generator with detachable filter of claim 1, wherein the humidifying module further comprises a gas channel extending upward from the bottom to the top of the humidifying module, the gas channel and the humidifying module are isolated from each other, and the filter channel device passes through the gas channel.

3. The hydrogen generator with detachable filter of claim 2, wherein the integrated channel device comprises a hole, and the filter channel device passes through the hole.

4. The hydrogen generator with detachable filter of claim 3, wherein the length of the filter channel device is larger than the length of the hole combined with the gas channel, and the filter channel device penetrates the integrated channel device and directly connects to the condenser.

5. The hydrogen generator with detachable filter of claim 1, wherein the condenser is detachably engaged with the integrated channel module, the condenser comprises a base engaged with the integrated channel module, a condensation tube detachably engaged with the base, and a heat dissipation element covering the condensation tube.

6. The hydrogen generator with detachable filter of claim 5, wherein the condenser further comprises a spiral structure configured in the condensation tube to form a condensation flow channel in the condensation tube, the gas comprising hydrogen passes through the condensation tube along the condensation flow channel.

7. The hydrogen generator with detachable filter of claim 5, wherein the length of the base of the condenser accounts for at least one-half of the side length of the hydrogen generator.

8. The hydrogen generator with detachable filter of claim 1, wherein the filter channel device further comprises a metal mesh component configured in the channel case.

9. The hydrogen generator with detachable filter of claim 1, wherein the filter component is a plurality of baffle structures, and the baffle structures are staggered and configured in the channel case to form a filter channel.

10. The hydrogen generator with detachable filter of claim 9, wherein the each baffle structure comprises an arc part and a bottom hook part connected to the arc part, wherein the arc part extends upward and the bottom hook part extends downward from the top of the arc part.

11. The hydrogen generator with detachable filter of claim 9, wherein the filter channel comprises a S-shaped flow channel.

12. The hydrogen generator with detachable filter of claim 1, further comprising a fining device, configured in the humidifying chamber and fluidly connected to the condenser through the integrated channel device, the fining device is configured to fine and evenly distribute the gas comprising hydrogen from the condenser into the humidifying chamber, wherein the fining device further comprises a plurality of micro holes for the gas comprising hydrogen flowing through into the humidifying chamber and forming a plurality of micro bubbles in the replenishing water.

13. The hydrogen generator with detachable filter of claim 1, wherein the electric potential of the water tank is equal to that of the filter channel device.

14. The hydrogen generator with detachable filter of claim 1, further comprising a nebulizer engaged with the integrated channel device, wherein the nebulizer receives the gas comprising hydrogen from the integrated channel device and selectively generates an atomized gas to be mixed with the gas comprising hydrogen to form a health caring gas.

15. The hydrogen generator with detachable filter of claim 1, further comprising an active filter tube penetrating the water tank and engaged with the humidifying module, the active filter tube being fluidly coupled to the humidifying module and the integrated channel device, and the active filter tube is detachable from the water tank; the active filter tube being configured to receive and filter the gas comprising hydrogen from the humidifying chamber and output the gas comprising hydrogen after filtering to the integrated channel device.

16. The hydrogen generator with detachable filter of claim 15, wherein the water tank comprises a tank body and a cover, the tank body comprises a tank channel and the cover comprises a cover channel, the active filter tube penetrates the cover channel and the tank channel to be connected to the humidifying module, wherein the gas comprising hydrogen flows from the humidifying module to the active filter tube, and then flows to the integrated channel module through the humidifying module.

17. The hydrogen generator with detachable filter of claim 1, further comprising a conductive component connected to the filter channel device and extending downward into the water tank.

18. A hydrogen generator with detachable filter, comprising:
a water tank, having an accommodation space for accommodating an electrolyzed water;
an electrolysis module configured to receive and electrolyze the electrolyzed water from the water tank and to generate and output a gas comprising hydrogen;
a humidifying module stacked above the water tank to humidify the gas comprising hydrogen, the humidifying module having a humidifying chamber to accommodate a replenishing water;
a filter channel device coupled with the water tank, the filter channel device being configured to receive and filter the gas comprising hydrogen from the electrolysis module and output the gas comprising hydrogen after filtering;
a condenser stacked above the water tank, the condenser being fluidly coupled to the filter channel device to receive and condense the gas comprising hydrogen from the filter channel device;
an integrated channel device stacked above the water tank, the integrated channel device being fluidly coupled to the condenser and the humidifying chamber to guide the gas comprising hydrogen from the condenser into the humidifying chamber; and
an active filter tube fluidly coupled to the humidifying module and the integrated channel device, the active filter tube being configured to receive and filter the gas comprising hydrogen in the humidifying chamber and output the gas comprising hydrogen after filtering to the integrated channel device;
wherein, the filter channel device, the condenser and the humidifying module are respectively engaged with the integrated channel device, the filter channel device penetrates the humidifying module and the integrated channel device, and the filter channel device is detachable from the humidifying module and the integrated channel module; the active filter tube penetrates the water tank and is engaged with the humidifying module, and the active filter tube is detachable from the water tank and the humidifying module.

19. The hydrogen generator with detachable filter of claim 18, wherein the electrolysis module is configured in the accommodation space of the water tank, the water tank comprises a tank body and a cover having a first fixing part, the electrolysis module having an electrolysis tank with a second fixing part toward the cover to be connected to the first fixing part, so as to suspend the electrolysis module from the cover.

20. The hydrogen generator with detachable filter of claim 19, wherein the cover comprises a first positioning structure, and the electrolysis module comprises a second positioning structure corresponding to the first positioning structure, wherein when the cover and the electrolysis module are connected to each other with the first fixing part and the second fixing structure to suspend the electrolysis module from the cover, the first positioning structure is coupled to the second positioning structure.

21. The hydrogen generator with detachable filter of claim 19, wherein the tank body forms a plurality of third positioning structures at the bottom of the accommodation space, and the bottom of the electrolysis module comprises a plurality of fourth positioning structures corresponding the third positioning structures, when the electrolysis module is configured in the accommodation space, the third positioning structures are respectively coupled with the fourth positioning structures.
